# EUROPEAN PATENT APPLICATION

(11) **EP 0 663 377 A1**
(43) Date of publication of application: **19.07.1995**
(21) Application number: 95100042.1
(22) Date of filing: 03.01.1995
(51) Int. Cl.: C07C 2/62

(54) **Alkylation process**

(30) Priority: 13.01.1994 DK 62/94
(71) Applicant: Haldor Topsoe A/S, DK-2800 Lyngby (DK)
(72) Inventor: Hommeltoft, Sven Ivar, DK-3400 Hillerod (DK)

(57) **Abstract**

Process for the alkylation of a hydrocarbon feedstock with an olefinic alkylating agent comprising the steps of contacting the hydrocarbon feedstock and the olefinic alkylating agent with a catalyst comprising a fluorinated alkane sulphonic acid having more than one carbon in the backbone or an acid mixture comprising at least one of the these for a sufficient time to ensure complete conversion and recovering a product stream of alkylated hydrocarbons.

## Description

It is well known that a number of strong acids catalyze the alkylation of isoalkanes with olefins to yield alkylate gasoline. Among the suitable catalysts are sulphuric acid, hydrogen fluoride and fluorinated sulphonic acids (cf. US pat. No. 5,220,095).

We have now found that when the alkylation reaction is performed using fluorinated alkanesulphonic acids with two or more carbons in the carbon chain as catalyst the product quality is of superior quality as compared to the product using trifluoromethanesulphonic acid especially at temperatures above 15°C. In particular, pentafluoro is suitable for ethane and heptafluoropropane sulphonic acids.

Accordingly, this invention provides a process for the alkylation of a hydrocarbon feedstock with an olefinic alkylating agent comprising the steps of contacting the hydrocarbon feedstock and the olefinic alkylating agent with a fluorinated alkane sulphonic acid catalyst with a chain length of two or more carbons for a sufficient time and recovering a product stream of alkylated hydrocarbons.

In a specific embodiment of the invention, the hydrocarbon feedstock is contacted with the catalyst in a fixed bed of solid contact material.

Suitable contact materials for use in the invention include non-basic refractory materials, preferably silica.

Presently it is preferred to carry out the process as a supported liquid phase process, wherein the catalyst is moveable supported on the contact material within a confined area thereof.

By the inventive process it is thus possible to produce a superior product at the same temperature, and to produce a product of comparable quality either at higher temperature and with a lower isobutane recycle. These possibilities represent economic advantages for the process.

The high alkylate quality as indicated by higher octane numbers are caused in part by a higher selectivity to C₈, but there is also a tendency towards more trimethylpentanes in the C₈-fraction.

### Example 1

A 100 ml reactor was packed with dried silica gel of the type Merck 100, 0.2-0.5 mm particle size. 6 ml CF₃SO₄H was pumped into the reactor and a feed stream containing 5% 2-butene in isobutane, and it was pumped through the reactor at a feed rate of 2.5 g/min. at temperatures varying in the range 0-30°C. The product composition was determined by GC and the octane numbers calculated from these compositions.
The results are shown in Table 1.

**Table 1**

| Temperature, °C | 0 | 10 | 20 | 30 |
|---|---|---|---|---|
| C₅₋₇ | 5 | 6 | 5 | 12 |
| C₈ | 89 | 85 | 82 | 75 |
| C₉₊ | 6 | 9 | 13 | 13 |
| TMP's in C₈ | 94 | 91 | 87 | 85 |
| RON | 99 | 98 | 96 | 95 |
| MON | 96 | 95 | 94 | 93 |

### Example 2

The same conditions as in Example 1 except that the acid used was C₄F₉SO₃H. The results are shown in Table 2.

**Table 2**

| Temperature, °C | 0 | 10 | 20 | 30 |
|---|---|---|---|---|
| C₅₋₇ | 4 | 3 | 5 | 6 |
| C₈ | 89 | 83 | 83 | 78 |
| C₉₊ | 8 | 13 | 12 | 16 |
| TMP's in C₈ | 93 | 91 | 89 | 88 |
| RON | 99 | 98 | 97 | 96 |
| MON | 96 | 95 | 94 | 93 |

### Example 3

A 100 ml reactor was packed with dried silica gel of the type Merck 100, 0.2-0.5 mm particle size. 6 ml perfluorinated ethanesulphonic acid (C₂F₅SO₃H) were introduced into the reactor. A feed stream containing 5% 2-butene in isobutane was then passed through the silica gel contact material at a feed rate of 2.5 g/min. at temperatures varying in the range 0-30°C. The product composition was determined by gas chromatographic analysis and the octane numbers calculated.
The results are shown in Table 3.

**Table 3**

| Temperature, °C | 0 | 10 | 20 | 25 | 30 |
|---|---|---|---|---|---|
| C₅₋₇ | 6 | 5 | 10 | 14 | 14 |
| C₈ | 89 | 87 | 83 | 78 | 77 |
| C₉₊ | 5 | 7 | 6 | 8 | 9 |
| TMP's in C₈ | 94 | 92 | 88 | 88 | 87 |
| RON | 99 | 99 | 97 | 96 | 96 |
| MON | 96 | 96 | 95 | 94 | 94 |

### Example 4

A 100 ml reactor was packed with dried silica gel of the type Merck 100, 0.2-0.5 mm particle size. 6 ml perfluoroethanesulphonic acid (C₂F₅SO₃H) were introduced into the reactor. A feed stream containing 5% 1-butene in isobutane was then passed through the silica gel contact material at a feed rate of 2.5 g/min. at temperatures varying in the range 0-30°C. The product composition was determined by gas chromatographic analysis and the octane numbers calculated from the obtained compositions.
The results are shown in Table 4.

**Table 4**

| Temperature, °C | 0 | 10 | 20 | 25 | 30 |
|---|---|---|---|---|---|
| C₅₋₇ | 6 | 6 | 9 | 11 | 15 |
| C₈ | 88 | 87 | 82 | 80 | 74 |
| C₉₊ | 6 | 7 | 9 | 9 | 11 |
| TMP's in C₈ | 93 | 91 | 88 | 87 | 84 |
| RON | 99 | 98 | 97 | 96 | 95 |
| MON | 96 | 95 | 94 | 94 | 93 |

### Example 5

A 100 ml reactor was packed with dried silica gel of the type Merck 100, 0.2-0.5 mm particle size. 6 ml perfluoroethane sulphonic acid (C₂F₅SO₃H) were introduced into the reactor. A feed stream containing 1.5% isobutene and 3.5% 2-butene in isobutane was then passed through the silica gel contact material at a feed rate of 2.5 g/min. and temperatures varying in the range 0-30°C. The product composition was determined by gas chromatographic analysis and the octane numbers estimated from these compositions.
The results are shown in Table 5.

**Table 5**

| Temperature, °C | 0 | 10 | 20 | 25 | 30 |
|---|---|---|---|---|---|
| C₅₋₇ | 10 | 13 | 14 | 15 | 17 |
| C₈ | 71 | 68 | 65 | 63 | 61 |
| C₉₊ | 20 | 19 | 21 | 22 | 22 |
| TMP's in C₈ | 92 | 89 | 87 | 86 | 84 |
| RON | 96 | 95 | 94 | 94 | 93 |
| MON | 94 | 93 | 93 | 92 | 92 |

### Example 6

A 100 ml reactor was packed with dried silica gel of the type Merck 100, 0.2-0.5 mm particle size. 6 ml perfluoroethane sulphonic acid (C₂F₅SO₃H) were introduced into the reactor. A feed stream containing 7.5% propene in isobutane was then passed through the silica gel contact material at a feed rate of 2.5 g/min. at temperatures varying in the range 0-30°C. The product composition was determined by gas chromatographic analysis and the octane numbers calculated.
The results are shown in Table 6.

**Table 6**

| Temperature, °C | 0 | 10 | 20 | 25 | 30 |
|---|---|---|---|---|---|
| C₅₋₇ | 42 | 45 | 50 | 52 | 52 |
| C₈ | 40 | 41 | 35 | 33 | 33 |
| C₉₊ | 18 | 14 | 15 | 15 | 15 |
| TMP's in C₈ | 88 | 87 | 84 | 82 | 79 |
| RON | 91 | 91 | 90 | 89 | 89 |
| MON | 89 | 89 | 88 | 87 | 86 |

### Example 7

A 100 ml reactor was packed with dried silica gel of the type Merck 100, 0.2-0.5 mm particle size. 6 ml perfluoropropane sulphonic acid (C₃F₇SO₃H) was introduced into the reactor. A feed stream containing 5% 2-butene in isobutane was then passed through the silica gel contact material at a feed rate of 2.5 g/min. at temperatures varying in the range 0-30°C. The product composition was determined by gas chromatographic analysis and the octane numbers calculated.
The results are shown in Table 7.

**Table 7**

| Temperature, °C | 0 | 10 | 20 | 25 | 30 |
|---|---|---|---|---|---|
| C₅₋₇ | 6 | 8 | 10 | 11 | 14 |
| C₈ | 89 | 86 | 82 | 80 | 77 |
| C₉₊ | 6 | 6 | 8 | 8 | 10 |
| TMP's in C₈ | 94 | 92 | 89 | 87 | 85 |
| RON | 99 | 99 | 97 | 96 | 94 |
| MON | 96 | 96 | 94 | 94 | 93 |

### Example 8

A 100 ml reactor was packed with dried silica gel of the type Merck 100, 0.2-0.5 mm particle size. 6 ml of a 1:1 mixture of trifluoromethanesulphonic acid (CF₃SO₃H) and nonafluorobutane 1-sulphonic acid (C₄F₉SO₃H) were introduced into the reactor. A feed stream containing 5% 2-butene in isobutane was then passed through the reactor at a feed rate of 2.5 g/min. at temperatures varying in the range of 0-40°C. The product composition was determined by gas chromatographic analysis and the octane numbers calculated.
The results are shown in Table 8.

**Table 8**

| Temperature, °C | 0 | 10 | 20 | 25 | 30 | 40 |
|---|---|---|---|---|---|---|
| C₅₋₇ | 4 | 7 | 10 | 11 | 13 | 20 |
| C₈ | 90 | 87 | 82 | 80 | 76 | 69 |
| C₉₊ | 6 | 6 | 8 | 9 | 11 | 11 |
| TMP's in C₈ | 94 | 92 | 88 | 86 | 83 | 79 |
| RON | 99 | 99 | 97 | 96 | 95 | 93 |
| MON | 96 | 96 | 95 | 94 | 93 | 92 |

## Claims

1. Process for the alkylation of a hydrocarbon feedstock with an olefinic alkylating agent comprising the steps of contacting the hydrocarbon feedstock and the olefinic alkylating agent with a catalyst comprising a fluorinated alkane sulphonic acid having more than one carbon in the backbone or an acid mixture comprising at least one of the these for a sufficient time to ensure complete conversion and recovering a product stream of alkylated hydrocarbons.

2. The process of claim 1, wherein the alkane sulphonic acid is perfluoro ethanesulphonic acid.

3. The process of claim 1, wherein the alkane sulphonic acid is perfluoro propanesulphonic acid.

4. The process of claim 1, wherein the alkane sulphonic acid is perfluoro 1-butanesulphonic acid.

5. The process of claim 1-4, wherein the hydrocarbon feedstock is contacted with the catalyst in a fixed bed of solid contact material.

6. The process of claim 1-4, wherein the solid contact material is selected from a group of non basic refractory materials, preferably silica.

7. The process of claim 1-4, wherein the catalyst is moveable supported on the contact material within a confined area thereof.

8. The process of claim 1-4, wherein the solid contact material comprises silica treated with boron phosphate or boron sulphate.
